Europäisches Patentamt

European Patent Office

Office européen des brevets

(19)

(11) EP 0 546 623 B1

(12) **FASCICULE DE BREVET EUROPEEN**

(45) Date de publication et mention
de la délivrance du brevet:
**24.09.1997 Bulletin 1997/39**

(51) Int Cl.6: **G01S 15/58, A61B 8/06**

(21) Numéro de dépôt: **92203769.2**

(22) Date de dépôt: **04.12.1992**

(54) **Echographe ultrasonore pour la mesure de vitesses élevées d'écoulements sanguins**

Ultraschallechograph zur Messung hoher Geschwindigkeiten von Blutströmungen

Ultrasonic echograph for measuring high velocities of blood flows

(84) Etats contractants désignés:
**DE FR GB**

(30) Priorité: **11.12.1991 FR 9115374**

(43) Date de publication de la demande:
**16.06.1993 Bulletin 1993/24**

(73) Titulaires:
• **LABORATOIRES D'ELECTRONIQUE PHILIPS
S.A.S.
94450 Limeil-Brévannes (FR)**
Etats contractants désignés:
**FR**
• **Philips Electronics N.V.
5621 BA Eindhoven (NL)**
Etats contractants désignés:
**DE GB**

(72) Inventeurs:
• **Bonnefous, Odile
F-75008 Paris (FR)**
• **Goujon, Antoine
F-75008 Paris (FR)**

(74) Mandataire: **Pyronnet, Jacques et al
Société Civile S.P.I.D.
156, Boulevard Haussmann
75008 Paris (FR)**

(56) Documents cités:
EP-A- 0 225 667       DE-A- 3 701 786
US-A- 4 799 490       US-A- 4 955 386

## Description

La présente invention concerne un échographe ultrasonore pour la mesure de profils de points de vitesse d'écoulements sanguins tel que défini dans le préambule de la revendication 1.

L'invention trouve une application particulièrement avantageuse comme profilomètre et comme appareil de visualisation bidimensionnelle en couleurs, dans le domaine de l'exploration échographique d'écoulements sanguins dans les vaisseaux, notamment la détection précise de sténoses affectant des artères.

Le problème technique général à résoudre par tout dispositif de mesure de la vitesse d'organes en mouvement et d'écoulements sanguins est d'obtenir une estimation aussi exacte que possible de la vitesse axiale du mouvement étudié afin de réaliser, à l'aide de dispositifs de visualisation, des images précises des organes et des écoulements sanguins soumis à une exploration échographique ultrasonore.

Depuis plusieurs années, diverses solutions à ce problème technique ont été proposées. En ce sens, la demande de brevet européen n° 0 225 667 décrit un dispositif de mesure de la vitesse d'organes en mouvement et d'écoulements sanguins conforme au préambule qui utilise le fait que les signaux ultrasonores successifs rétrodiffusés par une cible en mouvement, lorsque l'émission est récurrente à la période de récurrence T, sont reliés par l'équation suivante :

$$S_{n+1}(t) = S_n(t-\tau) \qquad (1)$$

Ceci signifie que le signal n+1 est la réplique du signal n précédent à un décalage temporel $\tau$ près. Ce dernier représente le temps supplémentaire nécessaire à l'onde ultrasonore pour parcourir le trajet transducteur-cible-transducteur, d'un tir à l'autre. Autrement dit :

$$\tau = 2VT/C$$

où V est la vitesse de la cible et C la vitesse du son. On voit qu'une mesure de $\tau$ permet de mesurer la vitesse V cherchée.

La fonction d'intercollélation entre $S_n(t)$ et $S_{n+1}(t)$ définie par :

$$C_{n,n+1}(to,u) = \int_{to}^{to+W} S_{n+1}(t+u) S_n(t) dt$$

vérifie :

$$C_{n,n+1}(to,u) = C_{nn}(to,u-\tau)$$

Le temps to est lié à la profondeur d'exploration z par to = 2z/C et W est la fenêtre d'intégration.

La fonction $C_{nn}(to,u)$ est une fonction d'autocorrélation, et, de ce fait, est maximale pour u = o. Ainsi, une mesure du décalage temporel $\tau$, et donc de la vitesse V, peut se faire en cherchant pour quel paramètre u la fonction $C_{n,n+1}$ (to,u) est maximale). A cet effet, la fonction d'intercorrélation est échantillonnée, à un pas d'échantillonnage $\Delta t$, entre $u_{min} = -l\Delta t$ et $u_{max} = l\Delta t$ par pas de 1 de façon à obtenir 2l+1 valeurs de fonctions de corrélation. La valeur maximum de ces 2l+1 valeurs correspondant à u = uo permet de mesurer $\tau$ en utilisant l'égalité $\tau$ = uo.

Afin de s'affranchir des erreurs inhérentes à l'échantillonnage dans la détermination du maximum de la fonction de corrélation, il est possible d'utiliser un circuit de multiplexage-interpolation fournissant à partir des valeurs des fonctions de corrélation une estimée plus précise de la vitesse et la valeur du pic de corrélation correspondant. La demande de brevet FR 2 590 790 au nom de la demanderesse donne un exemple de ce type de traitement du signal échographique dans lequel la corrélation entre signaux est une corrélation dite "1 bit" en ce sens que les signaux $S_{n+1}$ et $S_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

Cette méthode connue de mesure des vitesses, reposant sur l'analyse du décalage temporel, présente de sérieux avantages sur d'autres méthodes fondées, par exemple, sur le décalage de fréquence ou de phase. En particulier, elle permet d'utiliser des signaux d'émission à bande large, produisant une bonne résolution axiale de la mesure.

Toutefois, la méthode exposée ci-dessus ne permet pas de mesurer des vitesses supérieures à une vitesse limite $V_{lim}$ donnée par :

$$V_{lim} = \frac{C}{4} \frac{1}{f_0 T} \qquad (2)$$

où C représente la vitesse de propagation de l'onde ultrasonore. Ce phénomène connu aussi sous le terme d' "aliasing" est lié à l'indétermination induite par la périodicité du signal échographique. Une description détaillée en est donnée dans l'ouvrage "Doppler Ultrasound and Its Use in Clinical Measurement", P. Atkinson and J.P. Woodcock, Academic Press, 1982.

A titre d'exemple, avec une période de récurrence T de 100 μs, une fréquence acoustique centrale $f_0$ de 5 MHz et une vitesse de propagation C de 1500 m/s, on obtient une vitesse limite $V_{lim}$ de 75 cm/s, alors que certains écoulements sanguins, par exemple, peuvent atteindre des vitesses sensiblement supérieures.

Pour augmenter la vitesse limite de mesure, on pourrait penser à diminuer la fréquence $f_0$, mais cela conduirait à une réduction de la précision de la mesure et de la résolution. De même, une augmentation de la fréquence de récurrence aurait pour conséquence indésirable de diminuer la profondeur d'exploration.

Aussi, le problème technique à résoudre par l'objet de la présente invention est de réaliser un dispositif de mesure de la vitesse d'écoulements sanguins, du type de celui décrit dans le préambule, dispositif qui permettrait d'augmenter la vitesse limite de mesure $V_{lim}$ sans diminuer la fréquence $f_0$ et sans augmenter la fréquence de récurrence 1/T.

Indépendamment du phénomène d' "aliasing", un but de l'invention est de lever d'autres ambiguïtés, liées à l'échantillonnage, dans la détermination du pic de corrélation. Il peut se produire en effet que le point le plus haut de la fonction de corrélation échantillonnée n'appartienne pas au pic de corrélation recherché. Cette situation peut survenir lorsqu'on mesure des flux complexes, comportant des gradients de vitesse importants qui tendent à faire baisser le pic de corrélation. Cette erreur se traduit par des discontinuités brutales dans la reconstitution du profil de la vitesse en fonction de la profondeur d'exploration.

Une solution au problème technique posé, connue de la demanderesse, consiste en ce que ledit dispositif comporte une deuxième voie de traitement du signal échographique reçu, comprenant :

- deux filtres passe-bande symétriques $F_1$ et $F_2$ appliqués au signal $S_n(t)$, disposés en parallèle et fournissant respectivement des signaux $S_{n1}(t)$ et $S_{n2}(t)$ centrés respectivement autour d'une fréquence $f_1$ au plus égale à $f_0$ et d'une fréquence $f_2$ au moins égale à $f_0$, la différence $f_2-f_1$ étant inférieure à $f_0$,
- un multiplieur réalisant le produit des signaux $S_{n1}(t)$ et $S_{n2}(t)$,
- un filtre passe-bas symétrique sélectionnant la composante $S_n(t)$ à la fréquence $f_2-f_1$ du produit $S_{n1}(t) \times S_{n2}(t)$ , ,
- un deuxième circuit d'intercorrélation délivrant $2l+1$ valeurs échantillonnées de la fonction de corrélation de deux signaux $S_n(t)$ et $S_{n+1}(t)$ successifs, dite deuxième fonction de corrélation,

et en ce qu'un circuit de multiplexage-interpolation fournit une estimée de la vitesse par recherche du maximum de la première fonction de corrélation autour de l'échantillon donnant la valeur la plus grande de la deuxième fonction de corrélation.

Ainsi, ce dispositif connu met en jeu non seulement un signal $S_n(t)$ haute fréquence ($f_0$) comme le dispositif antérieur de l'état de la technique, mais aussi un deuxième signal $S_n(t)$ basse fréquence ($f_2-f_1$) qui vérifie également la relation (1) et qui peut donc être traité comme le signal $S_n(t)$. La deuxième fonction de corrélation, liée au signal $S_n(t)$, a une fréquence beaucoup plus faible et ne présente, dans le domaine de mesure considéré, pratiquement qu'un seul maximum, ce qui permet de lever totalement l'indétermination due au phénomène d' "aliasing" dans la mesure de la vitesse à partir de la première fonction de corrélation. Ce dispositif combine les avantages d'une précision de mesure élevée, déterminée par le signal à fréquence $f_0$ et d'une vitesse limite plus grande, imposée par le signal basse fréquence et dont la valeur est donnée par :

$$V_{lim} = \frac{C}{4} \frac{1}{(f_2-f_1)T}$$

La solution connue indiquée aux paragraphes précédents ne permet cependant pas d'obtenir des valeurs de vitesse limite très élevée. En effet, étant donnée la nécessaire séparation des filtres $F_2$ et $F_1$ destinés à sélectionner les fréquences $f_2$ et $f_1$, un rapport de fréquences $f_0/f_2-f_1$ de l'ordre de 2 à 3 seulement peut être obtenu, ce qui permet de passer d'une valeur $V_{lim}$ de 0,8 m/s à une valeur de l'ordre de 2 m/s. Ceci est suffisant pour mesurer toutes les vitesses de mouvement d'organes ou d'écoulements d'un corps humains en bonne santé mais pas pour mesurer certains flux

# EP 0 546 623 B1

sanguins relevant d'états pathologiques, notamment en cas de sténoses artérielles où le sang peut atteindre des vitesses de 5 m/s, ou des shunts artério-veineux présentant des vitesses sanguines encore supérieures.

Avec la solution connue indiquée ci-dessus, lorsqu'on cherche à rapprocher en fréquence les filtres $F_1$ et $F_2$, il faut leur conférer un front de coupure d'autant plus raide pour éviter qu'ils se recoupent, ce qui résulterait en une composante continue parasite dans leur signal de sortie. Ces filtres deviennent alors plus chers parce que plus longs, ce qui implique en outre l'inconvénient d'une perte en résolution et rend les corrélations moins précises.

Du brevet US-A 4 955 386 il est aussi connu, en technique d'échographie Doppler qui met en oeuvre des différences de fréquences et de phases, un étage d'émission qui comporte des premiers moyens pour émettre ledit signal impulsionnel selon deux périodes de récurrence voisines T et T + Ts alternées, et un circuit de suppression d'échos fixes (MTI) comportant des moyens de suppression adaptés à la période de récurrence somme 2T + Ts; deux chaînes de traitement de signaux Doppler permettent ensuite, par comparaisons de phases, de déduire une valeur non ambiguë de vitesse.

Selon l'invention, le problème technique précité est résolu et les limitations de l'art antérieur sont surmontées grâce à la mise en oeuvre des caractéristiques techniques donnés dans la partie caractérisante de la revendication 1.

Selon l'art antérieur, la profondeur maximale qu'il est possible de sonder est directement proportionnelle à la période de récurrence T. Selon l'invention, cette profondeur est limitée par la plus courte des deux périodes $T_1$ et $T_2$. De préférence, on choisit les valeurs de $T_1$ et $T_2$ proches de la valeur choisie habituellement pour T, par exemple telles que : $T_1<T<T_2$. La suppression d'échos fixes indispensable dans le cas d'un écoulement sanguin pour éliminer les échos de très forte amplitude dus aux parois du vaisseau implique une périodicité des instants d'émission (de tir) des impulsions. Cette périodicité égale à T, dans l'art connu, devient ici égale à la somme $T_1 + T_2$, quelle que soit la parité du tir d'une impulsion considérée. Cependant, à chaque parité (pair ou impair) correspond un signal de sortie particulier en sortie du circuit de suppression d'échos fixes et, en aval, à ces deux signaux correspondent deux corrélations séparées. La comparaison des deux fonctions de corrélation permet de lever toute ambiguïté qui serait due à l' "aliasing" ou à l' échantillonnage. On montre dans ces conditions que la vitesse limite qu'il est possible d'obtenir en vertu de la relation (2) ci-dessus est :

$$V_{lim} = \frac{C}{4} \frac{1}{f_0(T_2-T_1)}$$

Un compromis doit être fait pour le choix des valeurs de $T_1$ et $T_2$. Ces deux valeurs doivent être suffisamment distinctes et écartées l'une de l'autre pour que la présence d'un pic commun des deux fonctions de corrélation apparaisse à l'évidence et, en sens inverse, assez rapprochées pour obtenir un rapport $T/T_2-T_1$ assez élevé et donc aussi des valeurs de vitesses limites. Ce compromis n'est cependant pas critique et on peut par exemple choisir des valeurs de $T_1$, T et $T_2$ telles que :

$$T_1 = 5 T_0, \ T = 5,5 T_0 \ et \ T_2 = 6 T_0$$

La description qui suit en regard des dessins annexés, le tout donné à titre d'exemple non limitatif fera bien comprendre comment l'invention peut être réalisée.

La figure 1 est le schéma synoptique d'un échographe ultrasonore selon l'invention.

La figure 2 est le schéma synoptique de l'étage de réception et de traitement de la figure 1.

La figure 3 est un diagramme de temps qui représente le cadencement d'émission des impulsions ultrasonores selon l'invention.

La figure 4 montre en a et b des exemples de fonctions de corrélation obtenues au moyen de l'échographe de la figure 1 et en c comment ces fonctions sont comparées pour la mesure de chaque point de vitesse recherché.

Les figures 5 et 6 sont les schémas synoptiques de deux modes de réalisation du circuit de suppression d'échos fixes adapté à l'invention.

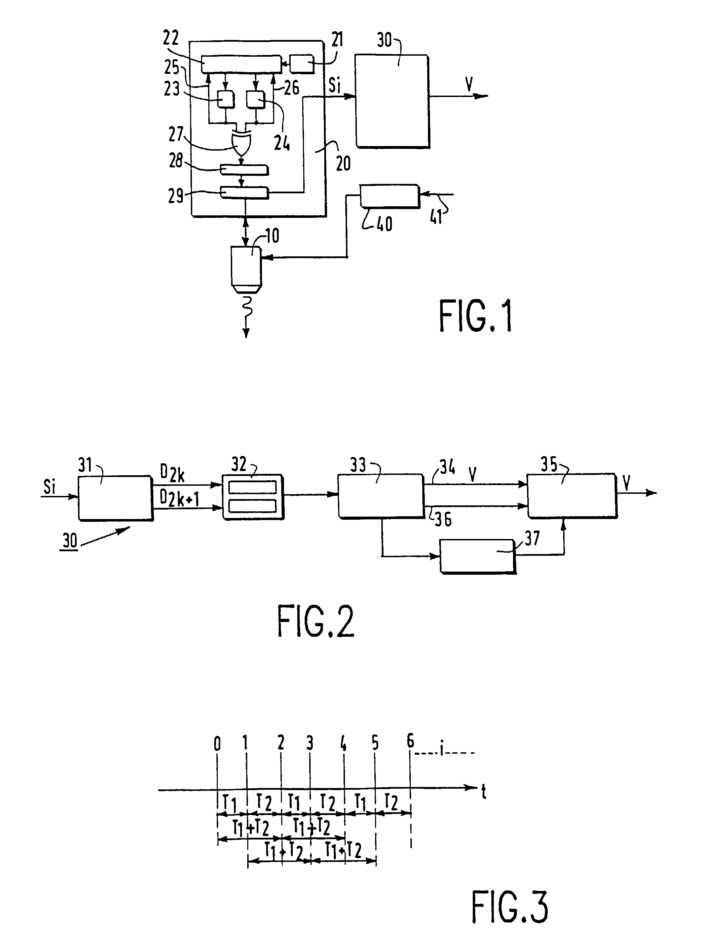

L'appareil d'exploration par échographie ultrasonore représenté à la figure 1 comprend, de façon classique, un transducteur ultrasonore 10 associé d'une part à un étage d'émission 20 et d'autre part à un étage 30 de réception et de traitement, ainsi qu'à un dispositif 40 de commande de balayage mécanique du transducteur. A la place de ce transducteur unique est utilisé, avantageusement, un réseau de transducteurs, associé alors à un dispositif de commande de balayage électronique. L'étage d'émission 20 comprend un générateur de signaux électriques d'excitation, envoyés vers le transducteur 10 qui, dans l'art connu, les convertit en trains périodiques de signaux impulsionnels ultrasonores, l'émission étant commandée par des signaux d'horloge délivrés à une fréquence de récurrence : F = 1/T déterminée - de l'ordre de 5 kilohertz par exemple - par un séquenceur interne au transducteur 10. Selon l'invention, l'étage d'émission 20 comporte des premiers moyens pour émettre les trains de signaux impulsionnels selon deux

4

périodes de récurrence voisines $T_1$ et $T_2$ alternées. Ces premiers moyens, dont la conception est, en soi, à la portée de l'homme de l'art, sont par exemple constitués par un générateur d'horloge 21 qui fournit un signal carré de fréquence $1/T_0$, par exemple : $1/T_0 = 27{,}5$ kHz, à un circuit logique 22 dont les deux sorties sont reliées à deux compteurs respectifs 23 et 24. Les compteurs 23 et 24 sont bouclés sur eux-mêmes et comptent le premier 23 jusqu'à n et le second 24 jusqu'à p (par exemple n = 5 et p = 6). La sortie du compteur 23 est reliée à une deuxième entrée 25 du circuit logique 22 et la sortie du compteur 24 à une troisième entrée 26 du circuit 22. Une impulsion reçue sur l'entrée 25 a pour conséquence de désactiver le compteur 23 pour activer le compteur 24 et une impulsion reçue en 26 a l'effet inverse. D'autre part ces sorties des compteurs sont reliées chacune à une entrée d'une porte OU exclusif 27 dont la sortie est reliée à l'entrée d'un générateur 28 de signaux électriques d'excitation. Les compteurs 23 et 24 sont par exemple du type 74F 160A ou du type 74F 168, fabriqués par la société Philips. Le signal de sortie du générateur 28 est envoyé, par l'intermédiaire d'un séparateur 29, vers le transducteur 10 qui les convertit en trains périodiques de signaux impulsionnels ultrasonores, de l'ordre de 4 à 10 impulsions. Le séparateur 29 de l'étage d'émission 20 et de l'étage de réception et de traitement 30, inséré de façon classique entre le transducteur 10, le générateur 28, et ledit étage 30, évite l'aveuglement des circuits de réception par les signaux d'émission. Avec les valeurs choisies ci-dessus : $T_0 = 36{,}4$ µs ; n = 5 et p = 6, on obtient comme périodes de récurrence alternées des impulsions, les valeurs : $nT_0 = 182$ µs ($1/T_1 = 5{,}5$ kHz) et $pT_0 = 218$ µs ($1/T_2 = 4{,}58$ kHz). Ces valeurs de période sont à comparer à celle couramment adoptée pour T dans la technique connue : T = 200 µs ($1/T = 5$ kHz).

Le dispositif 40 est commandé par un signal d'horloge, sur une entrée 41, à fréquence par exemple égale à 1 kHz. Dans le cas le plus fréquent où l'organe 10 est un réseau de transducteurs, le dispositif 40 a pour fonction de créer, à l'émission, une loi de distribution, d'un transducteur élémentaire à l'autre, de façon à focaliser en profondeur l'onde ultrasonore selon une droite en substance perpendiculaire au milieu (au corps) exploré.

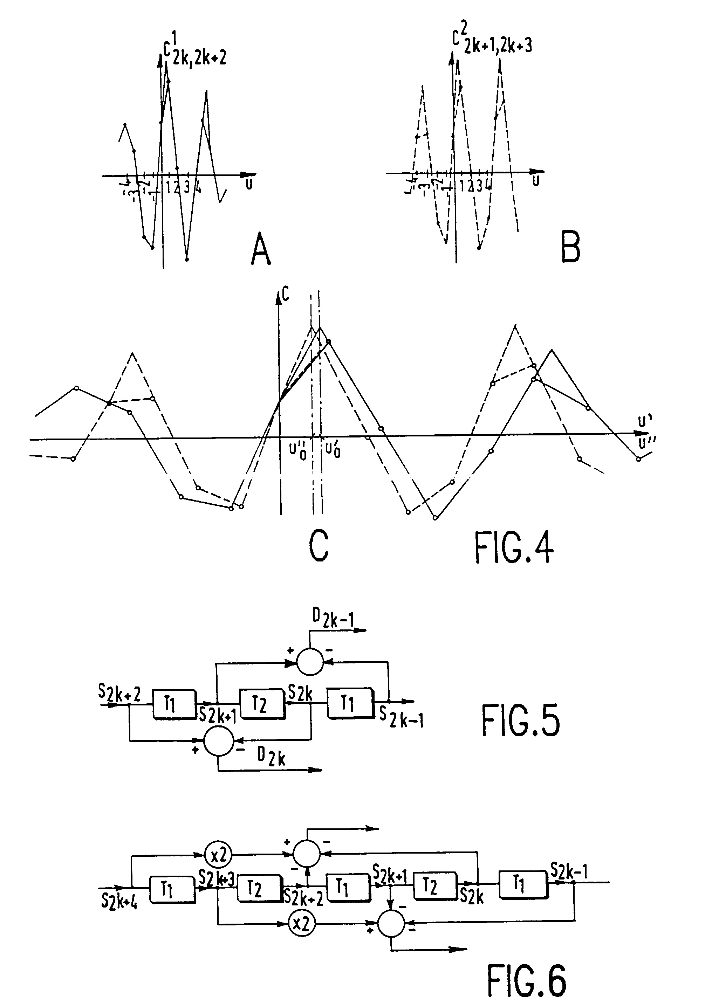

De façon connue l'étage de réception et de traitement 30 comprend, en sortie du séparateur 29, un amplificateur haute fréquence (non représenté) incluant la compensation de gain en fonction de la profondeur, suivi d'une voie de traitement comportant en cascade un circuit de suppression d'échos fixes, une mémoire d'échantillons numériques, un circuit de corrélation-interpolation, et un circuit de validation. Selon l'invention, cette voie de traitement représentée à la figure 2, présente certaines particularités. Le circuit de suppression d'échos fixes 31, toujours nécessaire à cause de l'amplitude très élevée des échos sur les parois des vaisseaux sanguins, doit comporter des deuxièmes moyens adaptés à la double période de récurrence $T_1$ et $T_2$ comme décrit ci-dessous en référence aux figures 5 et 6.

La sortie du circuit 31 n'est plus unique comme dans la technique connue, mais double et, pour des échantillons d'entrée $S_i$, les échantillons de sortie, débarrassés des échos fixes, ne sont pas des échantillons $D_i$ mais des échantillons de sortie pairs par exemple sur une sortie, soit $D_{2k}$ (pour : i = 2k) et impairs sur l'autre, soit $D_{2k+1}$ (pour : i = 2k+1). Sur une sortie apparaissent donc des échantillons qui sont relatifs à la période $T_1$ et sur l'autre des échantillons relatifs à la période $T_2$. La mémoire 32 de stockage d'échantillons (figure 2) est une mémoire double qui comporte, pour un train d'impulsions donné, dans une première moitié les échantillons $D_i$ des tirs pairs, associés à la période de récurrence $T_1$ par exemple, et dans une deuxième moitié les échantillons $D_i$ des tirs impairs, associés à la période de récurrence $T_2$.

Sur le diagramme de temps de la figure 3 est représentée de façon schématique l'émission d'un train d'impulsions. Sur l'axe des temps est indiqué l'instant qui marque le début de chaque impulsion, cet instant étant repéré par le numéro d'ordre de l'impulsion dans le train : 0, 1, 2, ..., i, ... ou en faisant intervenir la parité : 0, 1, 2, ..., 2k, 2k+1, ... Par exemple, comme représenté, les impulsions de numéro d'ordre pair marquent le début d'une période $T_1$ entre deux impulsions successives et ceux d'ordre impair le début d'une période $T_2$. Dans l'art connu, chaque train comporte environ N = 10 impulsions (10 tirs) qui servent à effectuer une moyenne sur N - n valeurs pour chaque point de vitesse, n étant le nombre de coefficients du filtre éliminateur d'échos fixes. Selon l'invention, étant donné que cette moyenne est effectuée deux fois sur N/2 - n valeurs, comme il ressort de la description qui suit, il est avantageux d'accroître le nombre N en le portant par exemple à 15. Il ne faut cependant pas augmenter trop la valeur de N sous peine de diminuer la cadence d'image pour le cas où le profil de vitesse mesuré est visualisé sur un moniteur.

Le circuit d'intercorrélation-interpolation, 33, fonctionne sur la base d'une corrélation "1 bit" comme décrit en détail dans la demande de brevet français précitée 2 590 790. Selon l'invention, le circuit 33 comporte deux ensembles corrélateur 1 bit. Le circuit d'intercorrélation connu comprend, de façon classique, une ligne à retard d'une période de récurrence T qui permet de recevoir en même temps deux signaux consécutifs $D_i(t)$ et $D_{i+1}(t)$. Dans le circuit 33 un ensemble corrélateur traite, au moyen d'une ligne à retard d'une période de récurrence $T_1$ deux signaux consécutifs pairs $D_{2k}(t)$ et $D_{2k+2}(t)$ (respectivement impairs) et l'autre ensemble corrélateur traite, au moyen d'une ligne à retard d'une période de récurrence $T_2$ deux signaux consécutifs impairs $D_{2k+1}(t)$ et $D_{2k+3}(t)$ (respectivement pairs), c'est-à-dire qu'un corrélateur traite, pour une première fonction de corrélation, deux lignes successives de la première moitié de la mémoire 32, chaque ligne correspondant à un tir et l'autre corrélateur, pour une deuxième fonction de corrélation, deux lignes successives de la deuxième moitié. Pour la fourniture des signaux-échantillons au circuit 33, l'adressage en lecture de la mémoire 32 peut être conçu séquentiellement, avec une seule sortie de données, par lecture d'une ligne d'un rang donné de la première moitié suivie de la lecture de même rang de la deuxième moitié, comme représenté à la figure 2, ou bien simultanée sur deux sorties drainant chacune une moitié de la mémoire. Dans chaque corrélateur, $2l + 1$ lignes à retard décalent respectivement l'un des deux signaux par rapport à l'autre de la quantité $u_j = j\Delta t$ j étant un nombre entier prenant les valeurs $- l, - l + 1, ..., - 1, 0, 1, ..., l - 1, l$ et $\Delta t$ le pas d'échantillonnage, par exemple 50 ns. Enfin, des corrélateurs au nombre de $2l + 1$ fournissent $2l + 1$ valeurs échantillons de chaque fonction de corrélation, ce qui définit une fenêtre de corrélation, soit :

$$C^1_{2k,2k+2}(t_0, uj)$$

et

$$C^2_{2k+1,2k+3}(t_0, uj)$$

j appartient à $[-l, l]$

Un exemple de telles fonctions de corrélation est donné aux figures 4a et 4b avec l supérieur à 4. A partir de ces deux fonctions, un point de vitesse recherché est obtenu pour la valeur d'abscisse $u_0 = \tau$ correspondant au pic de corrélation maximum (pic principal), cette valeur étant théoriquement la même pour les deux fonctions. En pratique, chaque fonction de corrélation est stockée et moyennée, pour une meilleure immunité au bruit, avec les suivantes qui correspondent aux paires de tirs suivantes pour la même profondeur $z_0$ associée à $t_0$. Cependant, il peut se faire que le pic principal se distingue mal des pics secondaires pour plusieurs raisons : en premier lieu, la fonction de corrélation est centrée en fréquence et oscille dans le temps avec la fréquence moyenne du signal échographique. D'autre part, en présence de bruit, comme c'est toujours le cas, les signaux sont décorrélés et la valeur du pic de corrélation peut décroître jusqu'à être du même ordre que celle des pics secondaires créés par l'oscillation de la fonction de corrélation. Par ailleurs, l'échantillonnage de calcul de cette fonction peut aussi induire une ambiguïté sur le choix de l'échantillon maximal avant l'interpolation qui permet d'obtenir le maximum de chaque pic de corrélation.

Selon l'invention, la recherche du pic de corrélation ne se limite plus à une zone ayant la taille de la période du signal échographique (ce que traduit la relation (2) ci-dessus) et la comparaison des fonctions de corrélation des figures 4a et 4b permet de déterminer avec certitude lequel, parmi plusieurs pics apparemment possibles, est le pic principal de corrélation, comme décrit ci-dessous.

Les deux signaux de corrélation des figures 4a et 4b sont de même période que le signal émis mais correspondent à des fréquences de répétition d'impulsion (PRF) différentes. Par conséquent, les pics principaux de corrélation correspondent dans chaque signal à des décalages temporels (abscusse u) différents, soit : $\tau_1 = 2 \, V/C \, T_1$ et $\tau_2 = 2 \, V/C \, T_2$, V étant la valeur de vitesse recherchée. On unifie l'échelle des décalages temporels entre les deux corrélations en les dilatant d'un facteur p pour la première (abscisse u') et n pour la seconde (abscisse u"), et on les superpose comme représenté à la figure 4c. Ainsi chaque signal contient un pic de corrélation décalé par rapport à 0 d'une durée : $u'_0 \sim u"_0 \sim 2 \, V/C \, np \, T_0$, dont il est aisé de déduire la vitesse V. La sélection de ce pic s'effectue en retenant le pic commun aux deux corrélations, les autres pics ayant été décalés par la dilatation. De préférence, afin de ne considérer que les pics significatifs, un seuillage préalable à 50 % du maximum est réalisé sur les fonctions de corrélation. Pour être assuré que les pics autres que les pics principaux sont tous décalés deux à deux, il faut que les nombres n et p soient premiers entre eux. En effet, toutes les possibilités de mesure de $\tau_1$ et $\tau_2$ s'expriment par :

$$\hat{\tau}_1 = \tau_1 + q/f_0, \text{ soit : } \hat{\tau}_1 = \tau_1 \text{ modulo } 1/f_0$$

$$\hat{\tau}_2 = \tau_2 + r/f_0, \text{ soit : } \hat{\tau}_2 = \tau_2 \text{ modulo } 1/f_0$$

$\hat{\tau}_1$ et $\hat{\tau}_2$ désignant les valeurs estimées de $\tau_1$ et $\tau_2$ respectivement, et q, r, des nombres entiers. Par ailleurs :

$$\tau_1 = \alpha n \, T_0$$

$$\tau_2 = \alpha p \, T_0$$

avec : $\alpha = 2V/C$

en remplaçant $\tau_1$ et $\tau_2$ par leurs valeurs dans les relations précédentes, on en arrive à l'équation :

$$qp - rn = 0$$

Pour que cette équation n'ait qu'une solution (aux multiples de q et de r près) il faut :

$$q = n$$

$$r = p$$

avec n et p premiers entre eux.

Le modulo qui va être appliqué à $\tau_1$ et $\tau_2$ est donc :
$n/f_0$ et $p/f_0$ respectivement.

On en déduit (par exemple à partir de $\tau_2$) :

$$|\tau_2| \leq \frac{p}{2}\frac{1}{f_0}$$

$$\frac{2V}{C}\,p\,T_0 \leq \frac{p}{2}\frac{1}{f_0}$$

$$V \leq \frac{C}{4f_0T_0}$$

Ceci montre par comparaison avec la relation (2) que la vitesse limite qu'il est ainsi possible de mesurer n'est plus en relation avec $T_1$ ni $T_2$ (comme elle l'est avec T dans l'art connu) mais avec $T_0$, soit une vitesse limite multipliée par n (n étant le plus petit des deux nombres n et p). La partie droite du schéma de la figure 2 est classique et décrite dans la demande de brevet français 2 662 265 au nom de la demanderesse. Après détermination de chaque valeur de vitesse moyennée du profil analysé, cette valeur est transmise, sur un conducteur 34, à un circuit de validation 35, dit encore circuit de segmentation qui reçoit directement du circuit 33, sur un conducteur 36, la valeur du pic principal de corrélation moyenné et, par l'intermédiaire d'un circuit de calcul d'amplitude 37, une estimation de l'amplitude (énergie) du signal pour le point du profil considéré. La valeur de vitesse est validée et fournie en sortie du circuit 35 seulement si les valeurs des deux autres paramètres (corrélation et amplitude) dépassent des seuils préétablis.

L'élimination des échos fixes se fait traditionnellement par différences successives ou combinaisons plus complexes. Dans le cas d'une émission récurrente de période T, les signaux de réception successifs subissent le traitement suivant :

$$D_1 = S_2 - S_1 \qquad \text{(deux coefficients, 1 et - 1)}$$

$S_1$ étant retardé de T par rapport à $S_2$ qui est le signal reçu, ou encore, de façon plus affinée :

$$D_1 = 2\,S_1 - S_2 - S_3 \qquad \text{(trois coefficients, 2, - 1 et - 1)}$$

$S_1$ étant retardé de 2T, et $S_2$ de T, par rapport à $S_3$ qui est le signal reçu.

Dans ces deux cas les lignes à retard ont, chacune, un retard égal à la période de récurrence d'émission T. On notera, dans ces deux exemples que si la relation :

$$S_i(t) = S_{i-1}(t-\tau) \text{ est vraie,}$$

alors la relation :

$$D_i(t) = D_{i-1}(t-\tau) \text{ est elle aussi vérifiée.}$$

Ceci exprime que l'on peut extraire la valeur de $\tau$ par corrélation temporelle des signaux D dans lesquels les parties communes (fixes) des signaux S ont été éliminées. Selon l'invention, cette étape de suppression d'échos fixes doit être adaptée puisqu'on utilise une double récurrence. Dans la transformation de signal à effectuer il faut nécessairement conserver la relation de récurrence des signaux retardés les uns par rapport aux autres. Or on remarque qu'il existe une périodicité dite globale dans le schéma d'émission à deux récurrences alternées, égale à : $T_1 + T_2$, comme indiqué à la figure 3 et le fait qu'il y ait chevauchement, d'une période globale à la période globale suivante, complique mais ne fait pas obstacle à l'élimination des échos fixes.

Les deux relations récurrentes qui lient les signaux successifs sont :

$$S_{2k+1}(t) \, S_{2k}(t-\tau_1)$$

$$S_{2k}(t) = S_{2k-1}(t-\tau_2)$$

Les nouveaux signaux D débarrassés des échos fixes sont engendrés de la manière suivante, comme représenté à la figure 5, dans le cas d'une simple différence (deux coefficients) :

$$D_{2k}(t) = S_{2(k+1)}(t) - S_{2k}(t) = D_{2k-1}(t-\tau_2)$$

$$D_{2k-1}(t) = S_{2k+1}(t) - S_{2k-1}(t) = D_{2k-2}(t-\tau_1)$$

Le cas de trois coefficients, par exemple (2, - 1, - 1), est représenté à la figure 6 :

$$D_{2k}(t) = 2S_{2(k+2)}(t) - S_{2(k+1)}(t) - S_{2k}(t)$$

$$= D_{2k-1}(t-\tau_2)$$

$$D_{2k-1}(t) = 2S_{2k+3}(t) - S_{2k+1}(t) - S_{2k-1}(t)$$

$$= D_{2k-2}(t-\tau_1).$$

## Revendications

1. Echographe ultrasonore pour la mesure de profils de points de vitesse d'écoulements sanguins comprenant au moins un transducteur ultrasonore (10) associé à un étage d'émission (20) d'un signal impulsionnel récurrent et à un étage de réception (30) des signaux échographiques renvoyés vers le transducteur (10) et de traitement de ces signaux reçus, l'échographe comprenant un système traitement de signal dans le domaine temporel avec intercorrélation des signaux écho graphiques relatifs aux différents signaux impulsionnels récurrents et interpolation des fonctions d'intercorrélations calculées, l'échographe comprenant en outre une voie de traitement numérique composée successivement d'un circuit de suppression des échos fixes (31), une mémoire d'échantillons numériques (32), un circuit d'intercorrélation-interpolation (33) et un circuit de validation (35), caractérisé en ce que ledit étage d'émission comporte des premiers moyens (21 à 28) pour émettre ledit signal impulsionnel selon deux périodes de récurrence voisines $T_1$ et $T_2$ alternées, ledit circuit de suppression d'échos fixes (31) comporte des deuxièmes moyens de suppression adaptés à la période de récurrence somme $T_1 + T_2$ ainsi que deux sorties d'indice pair et impair respectivement, ledit circuit d'intercorrélation-interpolation comportant en outre des troisièmes moyens (D2k, D2k+1) pour effectuer, à chaque point de vitesse, deux intercorrélations distinctes associées l'une à la période $T_1$, l'autre à la période $T_2$, puis pour comparer entre elles ces deux intercorrélations pour en déduire une valeur nom ambiguë de vitesse (V), les périodes $T_1$ et $T_2$ étant telles que : $T_1 = nT_0$, $T_2 = pT_0$, n et p étant des nombres entiers premiers entre eux, et la comparaison effectuée par lesdits troisièmes moyens consistant à dilater en abscisses par p la fonction d'intercorrélation associée à $T_1$, par n la fonction d'intercorrélation associée à $T_2$, à superposer les deux fonctions dilatées ainsi obtenues et à sélectionner, parmi les paires de pics de corrélation celle dont les deux abscisses du maximum sont les plus rapprochées.

2. Echographe ultrasonore selon la revendication 1 selon lequel : n = 5 et p = 6.

**Patentansprüche**

1. Ultraschallechograph zur Messung von Profilen von Geschwindigkeitspunkten von Blutströmungen mit wenigstens einem Ultraschallwandler (10), der zu einer Stufe (20) zur Aussendung eines sich wiederholenden Impulssignals und einer Stufe (30) zum Empfang der zum Wandler (10) zurückgesendeten Echographiesignale und zur Verarbeitung dieser empfangenen Signale gehört, wobei der Echograph ein System zur Signalverarbeitung im zeitlichen Bereich mit Korrelation der Echographiesignale, die sich auf verschiedene sich wiederholende Impulsignale beziehen, und zur Interpolation der berechneten Korrelationsfunktionen umfaßt, wobei der Echograph außerdem einen numerischen Übertragungskanal umfaßt, der nacheinander aus einer Festecho-Unterdrückungsschaltung (31), einem Speicher für numerische Abtastungen (32), einer Korrelations-Interpolationsschaltung (33) und einer Validierungsschaltung (35) besteht, dadurch gekennzeichnet, daß die genannte Stufe zur Aussendung erste Mittel (21 bis 28) umfaßt, um daß genannte Impulssignal in benachbarten, abwechselnden Wiederholungsperiodendauern $T_1$ und $T_2$ auszusenden, die genannte Festecho-Unterdrückungsschaltung (31) an die Wiederholungsperiodendauer-Summe $T_1 + T_2$ angepaßte zweite Unterdrückungsmittel sowie zwei Ausgänge mit geradzahligem bzw. ungeradzahligem Index umfaßt, wobei die genannte Korrelations-Interpolationsschaltung außerdem dritte Mittel (D2k, D2k+ 1) umfaßt, um bei jedem Geschwindigkeitspunkt, zwei verschiedene Korrelationen durchzuführen, von denen die eine zur Periodendauer $T_1$ gehört, und die andere zur Periodendauer $T_2$, um dann diese beiden Korrelationen miteinander zu vergleichen, um daraus einen nicht-mehrdeutigen Geschwindigkeitswert (V) abzuleiten, wobei die Periodendauern $T_1$ und $T_2$ so sind, daß $T_1 = nT_0$, $T_2 = pT_0$, wobei n und p teilerfremde ganze Zahlen sind, und der von den genannten dritten Mitteln durchgeführte Vergleich darin besteht, die zu $T_1$ gehörende Korrelationsfunktion bezüglich der Abszissen mit p und die zu $T_2$ gehörende Korrelationsfunktion mit n zu strecken, die beiden so erhaltenen gestreckten Funktionen zu überlagern und unter den Korrelationsspitzenpaaren, das zu selektieren, dessen beiden Abszissen des Maximums am engsten beieinanderliegen.

2. Ultraschallechograph nach Anspruch 1, bei dem gilt: n = 5 und p = 6.

**Claims**

1. An ultrasonic echograph for measuring velocity profiles of blood flows, comprising at least one ultrasonic transducer (10) which is connected to a stage (20) for the transmission of a recurrent pulsed signal and to a stage (30) for the reception of echographic signals returned to the transducer (10) and for processing the signals received, the echograph comprising a system for signal processing in the time domain with correlation of echographic signals relating to the various recurrent pulsed signals and interpolation of calculated correlation functions, the echograph also comprising a digital processing channel which is composed of successively a circuit (31) for the suppression of fixed echos, a digital sample memory (32), a correlation/interpolation circuit (33), and a validation circuit (35), characterized in that said transmission stage comprises first means (21 to 28) for transmitting said pulsed signal with two alternating, neighbouring recurrent periods $T_1$ and $T_2$, that said fixed echo suppression circuit (31) comprises second suppression means adapted to the sum recurrent period $T_1 + T_2$ as well as to outputs of even and odd index, respectively, that said correlation/interpolation circuit also comprises third means ($D_{2k}$, $D_{2k+1}$) for performing, at each velocity point, two distinct correlations, one of which is associated with the period $T_1$ and the other with the period $T_2$, and for subsequently comparing these two correlations with one another in order to deduce an unambiguous velocity value (V) therefrom, the periods $T_1$ and $T_2$ being such that $T_1 = nT_0$, $T_2 = pT_0$, n and p being mutual prime numbers, the comparison performed by said third means consisting of expanding in abscissae the correlation function associated with $T_1$ by p and the correlation function associated with $T_2$ by n, of superposing the two expanded functions thus obtained, and of selecting, from among the pairs of correlation peaks, that peak for which the two abscissae of the maximum are nearest one another.

2. An ultrasonic echograph as claimed in Claim 1, in which n = 5 and p = 6.

FIG.1

FIG.2

FIG.3

FIG.4

FIG.5

FIG.6